# EUROPÄISCHE PATENTSCHRIFT

(11) **EP 4 272 027 B1**
(45) Veröffentlichungstag und Bekanntmachung des Hinweises auf die Patenterteilung: **11.11.2026**
(21) Anmeldenummer: 21786838.9
(22) Anmeldetag: 30.09.2021
(51) Int. Cl.: G02B 21/16, C12Q 1/6825, G01N 21/64, G02B 21/30, G02B 21/36, G02B 27/56

(54) **OPTO-ELEKTRONISCHER CHIP**
OPTOELECTRONIC CHIP
PUCE OPTOÉLECTRONIQUE

(30) Priorität: 29.12.2020 DE 102020135024; 11.05.2021 DE 102021112251; 11.05.2021 DE 102021112256
(43) Veröffentlichungstag der Anmeldung: 08.11.2023
(73) Patentinhaber: Bruker Optics GmbH & Co. KG, 76275 Ettlingen (DE)
(72) Erfinder: TÜRSCHMANN, Pierre, 91052 Erlangen (DE); BÖNING, Daniel, 91052 Erlangen (DE)
(74) Vertreter: Lorenz Seidler Gossel Part. mbB
(86) Internationale Anmeldenummer: PCT/EP2021/076977
(87) Internationale Veröffentlichungsnummer: WO 2022/144101

(56) Entgegenhaltungen:
- WO-A1-2006/024314
- WO-A1-2009/141390
- WO-A2-01/53822
- US-A1- 2005 153 320
- US-A1- 2014 178 861
- BONINSEGNA MATTEO ET AL: "Waveguide chip coupled with microfluidics enables super-resolution live-cell imaging", SPIE PROCEEDINGS; [PROCEEDINGS OF SPIE ISSN 0277-786X], SPIE, US, vol. 11483, 21 August 2020 (2020-08-21), pages 114830T - 114830T, XP060133326, ISBN: 978-1-5106-3673-6, DOI: 10.1117/12.2568385

## Beschreibung

Die vorliegende Erfindung betrifft einen opto-elektronischen Chip zur Aufnahme einer Probe bei der Visualisierung temperatur-sensitiver Prozesse sowie ein optisches System mit einem derartigen Chip, insbesondere ein Mikroskop zur internen Totalreflexionsmikroskopie (TIRM) gemäß dem Oberbegriff des Anspruchs 1.

TIR-Mikroskopie kommt bevorzugt zur Untersuchung von Strukturen zum Einsatz, welche sich sehr nahe (zwischen 0 und 200 nm für sichtbares Licht) an einer Oberfläche, beispielsweise einer in Kontakt mit einer Probe stehenden Oberfläche eines Objektträgers, insbesondere eines opto-elektronischen Chips, befinden. Dies können beispielsweise fluoreszent markierte Moleküle oder Streuzentren in der Membran einer Zelle oder nahe an dieser, einzelne an die Oberfläche gebundene DNA-Moleküle oder auch andere Strukturen sein. Im Gegensatz zu klassischer Mikroskopie bietet TIR-Mikroskopie den Vorteil einer besseren Signalauflösung. Diese entsteht durch eine selektive Beleuchtung des oberflächennahen Bereichs des Deckglases in der klassischen TIR-Mikroskopie oder im speziellen im oberflächenahen Bereich des optisch-elektronischen Chips. Diese Beleuchtung wird durch ein in seiner Intensität von der Oberfläche exponentiell abfallendes Feld, auch evaneszentes Feld genannt, erzeugt. Dadurch wird ein hoher Kontrast zwischen dem oberflächennahen Signal und dem Hintergrundstreulicht generiert.

Herkömmlicherweise kommt bei der TIR-Mikroskopie ein Objektiv mit einer sehr hohen numerischen Apertur zum Einsatz, um zu gewährleisten, dass das Licht zur optischen Erregung der Probe mit einem flacheren Winkel als der kritische Winkel an der Grenzfläche zwischen dem Deckglas und der Probe totalreflektiert wird. Die Beleuchtungsgeometrie, im speziellen der exponentielle Abfall des evaneszenten Feldes, hängt unmittelbar mit dem Winkel zusammen, unter dem das Licht aus dem Objektiv austritt. Die Systeme zum Einstellen dieses Winkels sind jedoch sehr temperaturempfindlich und das Beobachtungsfeld ist auf eniger hundert µm² eingeschränkt. Sobald die Temperatur der Probe und somit oft auch des Objektivs um nur wenige Grad Celsius verändert wird, ändert sich die Probenbeleuchtung signifikant.

Da die TIR- Mikroskopie jedoch häufig zur. Untersuchung temperatursensitiver biologischer Prozesse eingesetzt wird (beispielsweise zur Bestimmung der Bindungsaffinität zwischen einem Protein und einem Antikörper oder von lebenden Zellen), ist es für den Erhalt verwertbarer Daten bei vielen Anwendungen essentiell, die Probe auf eine bestimmte Temperatur einzustellen.

Die Druckschrift US 2014/0178861A1 offenbart ein wellenleiterbasiertes optisches Erfassungsystem, welches beispielsweise für qPCR zum Einsatz kommen kann. Boninsegna Matteo et al., "Waveguide chip coupled with microfluidics enables superresolution live-cell imaging", SPIE Proceedings, DOI 10.1117/12.2568385 offenbart einen Mikrofluidik-Chip mit Temperierungsfluid zur Bildgebung lebender Zellen. Die WO 01/53822A2 offenbart grundsätzlich ein Verfahren zur Bestimmung temperatur-abhängiger Parameter. Die WO 2006/024314 A1 offenbart ein Verfahren zum Bestimmen von Punktmutationen mit sogenannten DNA-Microarrays und TIRF-Fluoreszenzanregung. Dabei wird die Bindung von Nukleinsäuren an kurze DNA-Sonden auf einem Microarray bei verschiedenen Temperaturen gemessen. Aus dem Messwerten werden Schmelzpunktkurven generiert und die Differenz der Schmelzpunktkurven zwischen der Sonde für die Wildtyp-DNA und der Sonde für die entsprechende mutierte-DNA generiert. Die Lage dieser Kurven lässt nun eine eindeutige Entscheidung zu, ob es sich um eine Homozygote DNA, oder eine heterzygote DNA für diese Punktmutation handelt und welche Art der Homozygotie vorliegt. Die US 2005/153320 A1 beschreibt, dass genetische Polymorphismen die Struktur und Funktion von Proteinen verändern können und damit die Veranlagung für Krankheiten und Gesundheitszustände beeinflussen. Ein Einzelnukleotid-Polymorphismus ist die kleinste genetische Mutation und am schwierigsten nachzuweisen. Allerdings machen Einzelnukleotid-Polymorphismen auch 90 % der bekannten genetischen Mutationen aus, weshalb die Identifizierung solcher Polymorphismen von entscheidender Bedeutung ist. Bei der Einzelbasen-Extension wird die Affinität einer Base zu ihrer komplementären Base genutzt, um Polymorphismen, einschließlich Einzelnukleotid-Polymorphismen, nachzuweisen. Planare Wellenleiter dienen als Plattform für die Einzelbasen-Extension und ermöglichen so den schnellen Nachweis genetischer Polymorphismen in Echtzeit. Es lassen sich Nachweisgrenzen im Pikomolarbereich erreichen. Die Signale der nicht passenden DNA-Basen liegen im Bereich des Blindsignals. Es werden Nachweiszeiten von 5 Minuten angegeben.

Die WO 2009/141390 A1 offenbart ein System zur Thermophorese bzw. einen opto-elektronischen Chip gemäß dem Oberbegriff des Anspruchs 1.

Vor diesem Hintergrund ist es eine Aufgabe der vorliegenden Erfindung, die Probleme des Stands der Technik abzumildern oder gar ganz zu beseitigen. Insbesondere liegt der vorliegenden Erfindung die Aufgabe zugrunde, eine Vorrichtung zu schaffen, welche die Notwendigkeit eines Objektives mit einer sehr hohen numerischen Apertur beseitigt und eine Einstellung der Probe auf eine gewünschte Temperatur zuverlässig und schnell ermöglicht als auch die Beobachtung größerer Beobachtungsfelder von bis zu einigen mm² ermöglicht,
Diese Aufgabe wird durch einen opto-elektronischen Chip mit den Merkmalen des Anspruchs 1 gelöst. Vorteilhafte Weiterbildungen der vorliegenden Erfindung sind Gegenstand der Unteransprüche.

Ein erfindungsgemäßer opto-elektronischer Chip dient zur Aufnahme einer Probe bei der Visualisierung temperaturabhängiger Prozesse und kann somit als Objektträger angesehen werden.

Ein derartiger opto-elektronischer Chip weist eine Trägerschicht, einen Lichtleiter (nachstehend auch als Wellenleiter bezeichnet), vorzugsweise einen Dünnschicht-Lichtleiter, und ein Dünnschicht-Heizelement, auf, wobei der Lichtleiter und das Heizelement vorzugsweise auf einander gegenüberliegenden Seiten der Trägerschicht angeordnet sind. Der Chip der vorliegenden Erfindung weist des Weiteren einen Temperatursensor auf. Dieser ist als Dünnschicht-Temperatursensor ausgebildet und in den Chip integriert.

Wird im Folgenden der Begriff Dünnschicht-Lichtleiter verwendet, so ist zu verstehen, dass dies lediglich eine bevorzugte Ausführungsform widerspiegelt und auch andere Lichtleiter von der Erfindung umfasst sind.

Das Heizelement und / oder der Lichtleiter ist / sind vorzugsweise optisch transparent.

Optisch transparentes Material ist hierbei vorzugsweise für Licht im für den Menschen sichtbaren Bereich eher durchlässig, wobei die Transmission des Lichts durch das optisch transparente Material vorzugsweise mindestens 0,5, insbesondere mindestens 0,8 beträgt. Optisch opakes Material ist hierbei vorzugsweise für Licht im für den Menschen sichtbaren Bereich eher undurchlässig, wobei die Transmission des Lichts durch das optisch opake Material vorzugsweise maximal 0,49, insbesondere maximal 0,3, beträgt.

Der Lichtleiter und / oder das Heizelement können unmittelbar an einer Oberfläche der Trägerschicht angeordnet sein oder von dieser über eine oder mehrere Zwischenschichten beabstandet sein.

Zudem können der Lichtleiter und / oder das Heizelement und / oder die Trägerschicht jeweils als eine einzige Schicht oder als Verbund zweier oder mehrerer Sub-Schichten ausgestaltet sein.

Vorzugsweise besteht die Trägerschicht vollständig oder zumindest teilweise aus einem opaken oder transparenten Material, vorzugsweise aus Si oder einem SiO₂ basiertem Glas oder Kristall.

Die Trägerschicht besteht somit beispielsweise aus Glas, insbesondere Borosilikatglass, und ist vorzugsweise dazu ausgelegt, dem opto-elektronischen Chip mechanische Stabilität zu verleihen.

Weiterhin kann sich zwischen der Trägerschicht und dem Dünnschichtwellenleiter eine weitere transparente Schicht befinden, die einen niedrigeren Brechungsindex als die Trägerschicht, vorzugsweise einen Brechungsindex zwischen 1.2 und 1.5, aufweist.

Gemäß einer Ausführungsform der Erfindung ist die Trägerschicht vollständig oder zumindest teilweise aus einem Halbleitermaterial, vorzugsweise aus Si, gefertigt und vorzugsweise ist weiterhin eine transparente Schicht, insbesondere eine Trennschicht, zwischen der Trägerschicht und dem Lichtleiter, vorzugsweise dem Dünnschicht-Lichtleiter, vorhanden.

Weiterhin ist das Dünnschicht-Heizelement mit einem Temperatur- Sensor in Form eines Dünnschicht-Temperatur-Sensors, verbunden und / oder ausgestattet, der in den Chip integriert ist. Dieser bildet vorzugsweise zumindest einen Teilbereich einer Oberfläche des opto-elektronischen Chips, welche dazu ausgelegt ist, mit einer Probe unmittelbar oder mittelbar in Kontakt zu treten.

Beispielsweise kann im Rahmen des Temperatursensors eine Sensor-Schicht zur Erfassung der Temperatur der Probe vorgesehen sein, welche vorzugsweise Metall aufweist und / oder aus Metall besteht und welche vorzugsweise eine Außenoberfläche des opto-elektronischen Chips zumindest teilweise bedeckt und weiterhin vorzugsweise dazu ausgelegt ist, mit einer Probe in Kontakt zu treten.

Vorzugsweise erfolgt die Messung der Temperatur mittels des Temperatursensors an mindestens einem Ort in der Probe, vorzugsweise an einer Mehrzahl an Orten, um einen verlässlicheren Messwert zu erhalten.

Vorzugsweise kommt im Rahmen des Temperatursensors eine Vierleitermessung zum Einsatz.

Weiterhin weist der opto-elektronische Chip vorzugsweise eine Steuerungseinheit auf, um das Dünnschicht-Heizelement auf der Grundlage der mittels des Temperatursensors erfassten Messdaten bezüglich der Probentemperatur zu steuern und / oder zu regeln.

Vorzugsweise ist ein im Rahmen der Erfindung zum Einsatz kommendes Dünnschicht-Heizelement ein Widerstandsheizelement oder umfasst ein solches. Beispielsweise können Karbon-Nanoröhren im Rahmen des Heizelements zum Einsatz kommen.

Um zu gewährleisten, dass zu untersuchende Partikel und / oder Objekte und / oder Moleküle nahe an einer Oberfläche des opto-elektronischen Chips und somit im Bereich der evaneszenten Wellen lokalisiert werden können, hat es sich in der Praxis als vorteilhaft erwiesen, wenn eine Außenoberfläche des opto-elektronischen Chips, welche dazu ausgelegt ist, mit der Probe in Kontakt zu treten, zumindest teilweise oder vollständig eine Oberflächenmodifikation und / oder Oberflächenfunktionalisierung aufweist, um in der Probe enthaltene Moleküle (oder sonstige Partikel und / oder Objekte), insbesondere biologische Moleküle, zu binden.

Eine Oberflächenfunktionalisierung kann beispielsweise ein Versehen der Oberfläche mit bestimmten funktionalen chemischen Gruppen, beispielsweise HydroxyGruppen, beinhalten, um eine gewünschte Molekülklasse gezielt an die Oberfläche zu binden.

Weiterhin betrifft die vorliegende Erfindung eine Verwendung eines erfindungsgemäßen opto-elektronischen Chips zur Aufnahme einer Probe bei der Visualisierung temperaturabhängiger Prozesse, wobei eine Probe, vorzugsweise eine zumindest teilweise flüssige, feste oder gelförmige Probe derart auf den opto-elektronischen Chip aufgebracht wird, dass die Probe den Dünnschicht-Lichtleiter und vorzugsweise auch die Sensor-Schicht des Temperatursensors teilweise oder vollständig bedeckt.Ein erfindungsgemäßer Chip kann zudem mit einem Mikrofluidik-System verwendet werden.

Beispielsweise kann ein erfindungsgemäßer opto-elektronischer Chip dazu verwendet werden, einen temperatur-sensitiven Prozess bei einer exakt kontrollierten Temperatur der Probe zu beobachten. Ein erfindungsgemäßer opto-elektronischer Chip kann weiterhin dazu verwendet werden, die Temperaturabhängigkeit eines Prozesses zu untersuchen, indem der Prozess bei unterschiedlichen exakt kontrollierten Temperaturen der Probe beobachtet wird.

Die im Rahmen der vorliegenden Erfindung eingesetzte Probe enthält vorzugsweise mindestens einen oder eine Mehrzahl an Partikeln und / oder Objekten und / oder Molekülen, welche dazu fähig und / oder ausgelegt ist / sind, mit einem geführten Modus (auch als Mode bezeichnet) des Dünnschicht-Lichtleiters in Wechselwirkung zu treten. Beispielsweise werden die Moleküle durch das von dem Lichtleiter geführte oder geleitete Licht zur Fluoreszenz angeregt, lenken dieses Licht ab und / oder absorbieren das Licht.

Ein weiterer Aspekt vorliegenden Offenbarung betrifft ein optisches System, vorzugsweise ein Mikroskop, besonders bevorzugt ein TIR-Mikroskop, welches dazu ausgelegt ist, mit einem erfindungsgemäßen opto-elektronischen Chip verwendet zu werden.

Ein optisches System gemäß der vorliegenden Offenbarung weist vorzugsweise mindestens einen Emitter, welcher Licht zur optischen Anregung in den Dünnschicht Wellenleiter entsendet und mindestens einen Detektor auf, welcher von der Probe abgelenktes und / oder ausgehendes Licht normal zu der Ebene des Dünnschicht-Lichtleiters erfasst.

Durch diesen Aufbau sind die Lichtpfade zur Anregung der Probe und zu Detektion des Lichts physisch voneinander getrennt, wodurch allgemeines Streulicht, das beim Einkoppeln des Lichts in den Wellenleiter oder bei der Führung des Lichts im Wellenleiter entsteht, und Streulicht aufgrund einer lokalen Streuung von Licht durch die Probe und Hintergrundlicht reduziert werden. Dies führt zu einem verbesserten Verhältnis zwischen den erwünschten detektierten Signalen aus der Probe gegenüber unerwünschter Signalen bedingt durch den Messaufbau.

Um Licht in einer für den Lichtwellenleiter typischen Mode in diesem zu führen, kommen vorzugsweise Kopplungsmodule, wie beispielsweise Gitterkoppler, Prismenkoppler und / oder direkte Kopplungsmechanismen zwischen zwei Lichtleitern zum Einsatz. Diese Kopplungsmodule dienen dazu externes Licht in den Wellenleiter einzuführen. Effizientere Kopplungsmodule können die Effizienz der Interaktion zwischen dem Emitter und dem Lichtleiter zu erhöhen.

Der Lichtleiter leitet das Licht des angeregten geführten Modus über den opto-elektronischen Chip und somit auch durch das Volumen der Probe.

Das von dem Lichtleiter geleitete Licht kann zurückreflektiert werden und / oder ausgekoppelt werden. Hierfür kommen vorzugsweise ebenfalls Kopplungsmodule, wie beispielsweise Gitterkoppler, Prismenkoppler und / oder direkte Kopplungsmechanismen zwischen zwei Lichtleitern zum Einsatz.

Ebenso ist es denkbar, dass mittels zusätzlicher Kopplungsmodule eine zeitgleich durch den Lichtleiter propagierende optische Mode einer anderen Wellenlänge, verschiedene optische Moden derselben Wellenlänge oder deren Kombination geführt werden. Eine Interaktion dieser innerhalb des Wellenleiters und deren Detektion kann für hochsensitive Messungen des Brechungsindex auf der Chipoberfläche verwendet werden.

Vorzugsweise ist bei einem optischen System gemäß der vorliegenden Offenbarung der Detektor ein Array-Detektor und / oder das optische System ist ein Mikroskop.

Weiterhin ist gemäß der vorliegenden Offenbarung eine Verwendung eines erfindungsgemäßen opto-elektronischen Chips und / oder eines genannten optischen Systems zur Bestimmung eines Phasenüberganges eines in der Probe enthaltenden (organischen oder eines anorganischen) Partikels oder eines räumlich ausgedehnten Materials. Dieser Phasenübergang kann beispielsweise die Veränderung eines biologischen Moleküls, beispielsweise eines Enzyms, eines Proteins oder einer Desoxyribonukleinsäure (DNA) oder Ribonukleinsäure (RNA) beinhalten, denkbar.

Ein anderer Aspekt gemäß der vorliegenden Offenbarung betrifft eine Verwendung eines erfindungsgemäßen opto-elektronischen Chips und / oder eines erfindungsgemäßen optischen Systems im Rahmen einer Hochdurchsatz-Sequenzierung, vorzugsweise auf der Grundlage der Analyse einzelner Moleküle.

Ein wieder anderer Aspekt gemäß der vorliegenden Offenbarung betrifft eine Verwendung eines erfindungsgemäßen opto-elektronischen Chips und / oder eines erfindungsgemäßen optischen Systems zur Untersuchung der Bindungsaffinitäten zwischen mindestens einem Protein und mindestens einem Antikörper in Abhängigkeit von der Temperatur.

Weiterhin ist gemäß der vorliegenden Offenbarung eine Verwendung eines erfindungsgemäßen opto-elektronischen Chips und / oder eines erfindungsgemäßen optischen Systems zur Untersuchung lebender Zellen unter temperatur-kontrollierten Bedingungen und deren Wechselwirkungen mit einzelnen Partikeln denkbar.

An dieser Stelle wird darauf hingewiesen, dass die Begriffe "ein" und "eine" nicht zwingend auf genau eines der Elemente verweisen, wenngleich dies eine mögliche Ausführung darstellt, sondern auch eine Mehrzahl der Elemente bezeichnen können. Ebenso schließt die Verwendung des Plurals auch das Vorhandensein des fraglichen Elementes in der Einzahl ein und umgekehrt umfasst der Singular auch mehrere der fraglichen Elemente.

Weitere Vorteile, Merkmale und Effekte der vorliegenden Erfindung ergeben sich aus der nachstehenden Beschreibung bevorzugter Ausführungsbeispiele unter Bezugnahme auf die Figuren. Hierbei zeigt:
Fig. 1 schematisch einen erfindungsgemäßen opto-elektronischen Chip;
Fig. 2 schematisch ein erfindungsgemäßes opto-elektronisches System;
Fig. 3 einen Querschnitt eines anderen erfindungsgemäßen opto-elektronischen Chips; und
Fig. 4 eine Draufsicht des erfindungsgemäßen opto-elektronischen Chips aus Fig. 3.

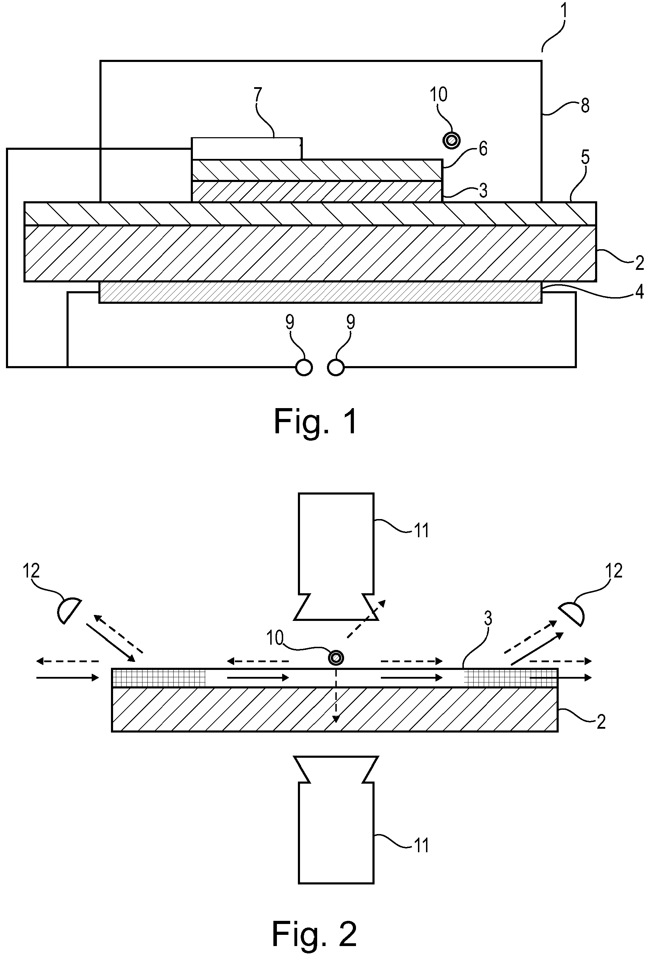

Fig. 1 zeigt einen opto-elektronischen Chip 1 gemäß der vorliegenden Erfindung. Der Chip 1 weist eine Trägerschicht 2, in dieser Ausgestaltung aus einem transparenten Silizium-basiertem Material, einen Dünnschicht- Lichtleiter 3 und ein Dünnschicht-Heizelement 4 auf. Der Dünnschicht- Lichtleiter 3 und das Dünnschicht-Heizelement 4 sind auf zwei gegenüberliegenden Seiten der Trägerschicht 2 angeordnet.

Zwischen der Trägerschicht 2 und dem Dünnschicht-Lichtleiter 3 ist eine Trennschicht 5 aus einem transparenten Material angeordnet.

Auf der der Trägerschicht 2 abgewandten Seite des Lichtleiters 3 ist eine weitere Trennschicht 6 angeordnet, welche zwischen dem Lichtleiter 3 und einer metallischen Sensor-Schicht 7, welche zur Erfassung der Temperatur der Probe 8 dient, positioniert ist. In der Probe 8 ist in dieser Darstellung ein Partikel 10 oder auch ein sonstiges Objekt, beispielsweise ein Molekül, enthalten.

Die Sensor-Schicht 7 ist in dieser Ausgestaltung mit einer nicht gezeigten Steuerungseinheit verbunden, welche das Heizelement 4 auf der Grundlage der mittels der Sensor-Schicht 7 erfassten Daten steuert und / oder regelt. Der elektronische Schaltkreis zur Steuerung und / oder Regelung des Heizelements 4 ist in Fig. 1 nur stilisiert dargestellt und mit dem Bezugszeichen 9 bezeichnet.

Das Heizelement 4 kann in einem Feedbackmodus betrieben werden, bei welchem der ausgelesene Wert des Temperatursensors, insbesondere der Sensor-Schicht 7, als Feedbackparameter verwendet wird. Die Regulierung findet vorzugsweise elektronisch statt. Es besteht auch die Möglichkeit, das Heizelement 4 nicht reguliert oder ohne eine Feedback-Regelung zu verwenden.

Fig. 2 zeigt ein optisches System, in welchem ein erfindungsgemäßer Chip 1 zum Einsatz kommt. Der Chip 1 ist in dieser Darstellung sehr vereinfacht wiedergegeben, dargestellt sind nur die Trägerschicht 2 und der Lichtleiter 3.

Licht wird von einem nicht gezeigten Emitter zur Anregung des Lichtleiters 3 in diesen eingekoppelt. In der Darstellung in Fig. 2 propagiert das Licht des angeregten geführten Modus (auch als "guided mode" bezeichnet) des Lichtleiters 3 von links nach rechts durch den Lichtleiter 3, wie dies in Fig. 2 durch die mit durchgängiger Linienführung dargestellten Pfeile gezeigt ist.

Der Partikel 10, welcher sich sehr nahe an der Oberfläche des Lichtleiters 3 befindet, kann mit dem Licht des propagierenden geführten Modus des Lichtleiters 3 interagieren, beispielsweise, indem der Partikel das Licht absorbiert oder von dem Licht zur Fluoreszenz angeregt wird. Beispielsweise kann durch den Partikel 10 das Licht abgelenkt, gestreut und / oder reflektiert werden, wie dies in Fig. 2 durch die mit strichlierter Linienführung dargestellten Pfeile gezeigt ist.

Derart von dem Partikel 10 beeinflusstes und abgelenktes Licht kann beispielsweise mittels Detektoren 11, z.B. Bildgebungssystemen, erfasst werden. Hierbei verläuft der Pfad des derart erfassten Lichts vorzugsweise normal, d.h. rechtwinklig, zu der Ebene des Dünnschicht- Lichtleiters 3 und somit auch normal bzw. rechtwinklig zu der Propagationsrichtung des angeregt geführten Modus des Lichtleiters 3.

Der Lichtpfad zur Anregung des Lichtleiters 3 (in Fig. 2 von links nach rechts) und der Lichtpfad zur Erfassung von Licht von der Probe 8 bzw. dem Partikel 10 (in Fig. 2 oben und unten) sind somit in dieser Ausgestaltung räumlich voneinander getrennt und verlaufen vorzugsweise normal, oder in anderen Worten orthogonal, zu einander.

In anderen Worten erfolgt somit eine räumlich aufgelöste Erfassung des von der Probe rechtwinklig zu der Ebene des Lichtleiters 3 abgelenkten Lichts von oben und / oder von unten durch die Trägerschicht des Chips 1. Das von der Probe rechtwinklig zu der Ebene des Lichtleiters 3 abgelenkte Licht kann beispielsweise rot-verschoben oder blau-verschoben relativ zu dem geführten Modus des Lichtleiters sein oder auch zu diesem resonant sein.

Die Probe und / oder darin enthaltene Partikel interagiert bzw. interagieren mit einer von dem geführten Modus des Lichtleiters herrührenden evaneszenten Welle, wodurch das Licht des geführten Modus beispielsweise gestreut, absorbiert oder mit einer anderen Wellenlänge re-emittiert wird.

Darüber hinaus kann mittels Photodetektoren 12 das in dem Lichtleiter 3 im geführten Modus geleitete resonante Licht erfasst werden und / oder das in den geführten Modus des Lichtleiters 3 gestreute Licht erfasst werden. Das in den geführten Modus des Lichtleiters 3 gestreute Licht ist beispielsweise rot-verschoben oder blau-verschoben relativ zu dem geführten Modus des Lichtleiters.

Ein typisches Vorgehen zur optischen Anregung und Detektion, wie es im Rahmen eines Systems gemäß Fig. 2 zum Einsatz kommt, wird nachstehend wiedergegeben:
Licht wird über ein Kopplungsmodul in die Wellenleitermode geschickt oder eingeleitet. Ein Anteil des Einkopplungslichts kann reflektiert werden, ein anderer Anteil des Lichts kann durch die Mode transmittiert werden. Das transmittierte Licht kann in einem zweiten Kopplungsbereich wieder gestreut werden.

Beide Anteile des Lichts können über einen Photodetektor detektiert werden und beispielsweise als Feedbacksignal oder Regelparameter zur Intensitätsstabilisierung des im Wellenleiter geführten Lichtanteils genutzt werden. Zur einer derartigen Intensitätsstabilisierung kann das im Einkopplungsbereich reflektierte Licht und / oder das im Auskopplungsbereich transmittierte Licht der Wellenleitermode als Feedbacksignal Anwendung finden, um die Intensität des Lichts im Wellenleiter zu stabilisieren bzw. kontrolliert zu verändern. Hierfür kann ein erfindungsgemäßer Chip eine entsprechend konfigurierte Steuerung aufweisen.

Licht kann über mehr als ein Einkopplungsmodul in den Wellenleiter eingekoppelt werden. Alternativ oder zusätzlich kann auch Licht mit einer unterschiedlichen Polarisation, Wellenlänge, Propagationsrichtung etc. gleichzeitig oder sequenziell eingekoppelt werden. Über die Kopplungsbereiche gestreutes Licht kann zudem zur Analyse des Probenvolumens herangezogen werden.

Ein Partikel 10 (bspw. Ein Biomolekül) kann mit der geführten Mode über den evaneszenten Lichtanteil wechselwirken (aktiver Probenbereich). Das durch den Partikel gestreute Licht (Fluoreszenz und / oder direktes Streulicht) kann über ein oder zwei optische Systeme oder Detektoren 11 örtlich aufgelöst detektiert werden, welche sich vorzugsweise oberhalb bzw. unterhalb des optoelektronischen Chips befinden. Durch den Partikel 10 gestreutes Licht kann auch in die Wellenleitermode einkoppeln und über die Kopplungsmodule gestreut und somit detektiert werden.

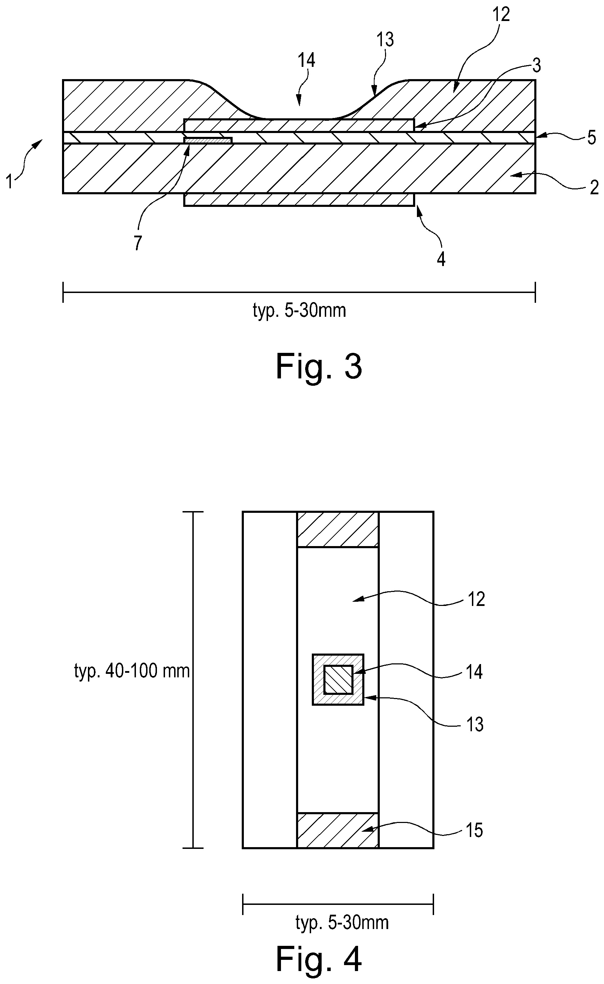

Fig. 3 zeigt eine weitere Ausführungsform der Erfindung. Insbesondere zeigt Fig. 3 einen Querschnitt eines opto-elektronischen Chips 1 mit einer beispielhaften Anordnung von Schichten und Schichtdicken. Der aktive Bereich 14 wird durch eine Schutzschicht 12 definiert, die in dieser Ausführungsform adiabatisch ausläuft (grundsätzlich sind auch nicht adiabatische Übergänge denkbar). Die adiabatische Überführung der Schutzschicht 12 ist mit dem Bezugszeichen 13 bezeichnet. Nur der aktive Bereich 14 des Chips 1 kommt mit einem Probenvolumen in Kontakt.

Die Schutzschicht 12 hat einen niedrigeren Brechungsindex als der Wellenleiter 3, vorzugsweise liegt der Brechungsindex der Schutzschicht im Bereich von 1.3 bis 1.5 im sichtbaren Bereich.

Die adiabatische Überführung 13 vom Schutzbereich bzw. einem von dem aktiven Bereich 14 abgewandten oder beabstandeten Bereich der Schutzschicht 12 hin zu dem aktiven Bereich 14 ermöglicht ein Modenübergang ohne eine Erzeugung von Streulicht und / oder einen Verlust von Lichtleistung unabhängig von einem Brechungsindex des Probenvolumens.

Weiterhin verhindert die Schutzschicht 12 das Auftreten von Verschmutzungen im Einkopplungsbereich und Streulicht durch einen etwaigen optionalen Behälter oder Kanal zur Aufnahme einer Probe auf oder in dem opto-elektronischen Chip. Bei der in Fig. 3 gezeigten Anordnung kann die Probe einfach in der durch den aktiven Bereich 14 gebildeten Mulde aufgenommen werden.

Das Heizelement 4 kann auf einer dem aktiven Bereich 14 entgegengesetzten Seite des Chips 1 angeordnet sein. Alternativ oder zusätzlich kann ein Heizelement 4 zwischen der Trennschicht 5 und der Trägerschicht 2 vorhanden sein. Bei einem Chip können auch mehrere Heizbereiche vorgesehen sein.

Der Temperatursensor oder zumindest dessen Sensor-Schicht 7 kann zwischen wischen der Trennschicht 5 und der Trägerschicht 2 angeordnet sein. Grundsätzlich kann der Sensor oder zumindest dessen Sensor-Schicht 7 auch an einer anderen Stelle des Chips 1 angeordnet sein, hier ist jedoch grundsätzlich darauf zu achten, dass sich der Temperatursensor und / oder ein Bestandteil davon nicht im evaneszenten Feld des Wellenleiters 3 befindet.

Der Temperatursensor bzw. die Sensor-Schicht 7, die Trennschicht 5 und die Schutzschicht 12 sind optional. Typische Schichtdicken der im Rahmen eines erfindungsgemäßen opto-elektronischen Chips zum Einsatz kommenden Schichten sind nachstehend wiedergegeben: Schutzschicht 12 (optional): 100 - 1000 nm, vorzugsweise 300-800nm, Wellenleiterschicht 3: 50- 1000 nm, vorzugsweise 75 - 250nm, Trennschicht 5 (optional): 100 - 1000 nm, vorzugsweise 100- 800nm, Trägerschicht 2: 150 - 1000 µm, vorzugsweise 170 - 500 µm, Heizelement 4: 5-100nm, vorzugsweise 10 - 50 nm. Der in Fig. 3 gezeigte Chip 1 hat eine Breite (Abmessung von rechts nach links in Fig. 3) von 5 - 30 mm.

Fig. 4 zeigt eine Draufsicht auf den optoelektronischen Chip aus Fig. 3. Der in Fig. 4 gezeigte Chip 1 hat eine Breite (Abmessung von rechts nach links in Fig. 4) von 5 - 30 mm und eine Länge (Abmessung von oben nach unten in Fig. 4) von 40-100 mm. Ein oder mehrere Kopplungsbereiche 15 ermöglichen das Ein- und Auskoppeln von Licht in die bzw. aus den geführten Wellenleitermoden. In der in Fig. 4 gezeigten Ausführungsform sind zwei Kopplungsbereiche 15 an einander gegenüberliegenden Enden des Chips 1 gezeigt.

Die Kopplungsbereiche 15 und der Wellenleiter 3 sind vorzugsweise von der Schutzschicht 12 überdeckt und nur in dem aktiven Bereich 14 ist die Wellenleiterschicht 3 exponiert und kann direkt mit dem Probenvolumen in Kontakt treten. Die Wellenleiterschicht 3 kann teilweise oder vollständig chemisch funktionalisiert sein. Der aktive Bereich 14 hat vorzugsweise eine Fläche von 0.01 mm² - 25 mm² und die Gesamtfläche des optoelektronischen Chips 1 beträgt vorzugsweise 25 mm²- 2000 mm².

Die vorliegende Erfindung bietet signifikante technische Vorteile, insbesondere die nachstehenden Vorteile:
Die vorliegende Erfindung ermöglicht die Betrachtung eines großen optischen Beobachtungsfelds. Insbesondere durch die Trennung von Anregungs- und Detektionspfad wird es technisch möglich, einen größeren Probenbereich bzw. aktiven Bereich optisch anzuregen. Konventionelle TIR-Systeme beleuchten Probenbereiche von typischerweise wenigen 10⁻³ mm², wohingegen die vorliegende Erfindung die optische Anregung von Flächen bis in den Bereich von einigen mm² ermöglicht. Dies eröffnet neue Möglichkeiten, hochparalellisiert eine große Anzahl von Biomolekülen zu detektieren. Darüber hinaus ermöglicht es die vorliegende Erfindung, komplexere biologische Systeme wie Zellen bzw. Zellhaufen optisch anzuregen und zu beobachten.

Darüber hinaus bietet ein erfindungsgemäßer optoelektronischer Chip im Vergleich zu konventionellen TIR-Systemen eine homogene Ausleuchtung des Probenbereichs bzw. aktiven Bereichs.

Auch das Signal-zu-Hintergrundverhältnis ist mit einem erfindungsgemäßen opto-elektronischen Chip verbessert. Ein derartiger optoelektronischer Chip bietet im Vergleich zu konventionellen TIR-Systemen einen stark verminderten Streulichthintergrund, da das anregende Lichtfeld nicht durch die Detektionsoptik geleitet wird.

Weiterhin bietet die vorliegende Erfindung den Vorteil, dass durch die geschickte Wahl der Wellenleiterschichtparameter und der Wellenlänge des Lichts die Eindringtiefe des evaneszenten Feldes in das Probenvolumen über einen großen Bereich variiert werden kann. Diese Eindringtiefe kann von einigen wenigen 10 nm bis hin zu mehreren 100 nm variieren, je nach Schicht- und Probeneigenschaften und Wellenlänge des Lichts.

Zudem ist durch die optische Anregung über die Wellenleitermode die Verwendung von Objektiven mit einer hohen numerischen Apertur nicht mehr erforderlich, da der nötige Einfallwinkel des Lichts durch die Wellenleitermode unterstützt wird. Auch die Verwendung von Immersionsmedium ist für das Erreichen der Bedingungen der totalen internen Reflexion nicht mehr nötig, was die Nutzerfreundlichkeit stark verbessert.

Weiterhin kann durch ein lokales Heizen des Probenvolumens mittels eines erfindungsgemäßen Chips die Temperatur der Probe sehr schnell eingestellt werden. Erfindungsgemäß sind Heizraten mit bis zu 100°C/s möglich. Da nur kleine Probenvolumina geheizt werden, ist die Wärmekapazität gering und bereits die Umgebung ermöglicht ein schnelles Abkühlen der Probe auf Umgebungstemperatur mit (Ab- )kühlraten von mehr als -20°C/s.

Herkömmliche Ansätze zur makroskopischen Temperaturregulierung ziehen diverse Nachteile mit sich, wie z.B. lange Equilibrierungszeiten, thermische Drifts, verschlechterte optische Abbildungseigenschaften etc., die durch die vorliegende Erfindung umgangen werden.

Darüber hinaus wird durch die Integration eines Dünnschichttemperatursensors in den opto-elektronischen Chip eine direkte Feedback-Regelung des Heizelements möglich. Auf diese Weise wird eine hochpräzise und dynamische Temperaturregulierung des Probenvolumens gewährleistet.

Die hohe Sensitivtität des optoelektronischen Chips bezüglich optischer Anregung und thermische Veränderungen ermöglicht darüber hinaus die calorimetrische Detektion von Phasenübergängen.

Durch die Integration einer hochsensitiven Anregung, eines Heizelements und eines Temperatursensors in den opto-elektronischen Chip, kann das Gesamtsystem zudem in Größe und Komplexität stark reduziert werden.

Da der opto-elektronische Chip über keine beweglichen Elemente verfügt, wird ein mechanischer Verschleiß bzw. eine Vibration des Gesamtsystems vermieden und die mechanische Stabilität des Systems optimiert.

Der Aufbau des opto-elektronischen Chips ist weiterhin grundsätzlich mit Mikrofluidikkanälen kompatibel. Insbesondere die Verwendung einer Schutzschicht mit adiabatischem Übergang zum aktiven Probenbereich gewährleistet die Funktionalität des Anregungssystems unabhängig von dem Brechungsindex des Probenvolumens, wenn dieser niedriger als der Modenindex des Wellenleiters ist, und / oder von einem potentiellen Probencontainer oder Kanal, welcher sich auf oder in dem optoelektronischen Chip befindet.

## Patentansprüche

1. Opto-elektronischer Chip (1) zur Aufnahme einer Probe (8) bei der Visualisierung temperatur-abhängiger Prozesse mittels eines Mikroskops, mit einer Trägerschicht (2), einem Dünnschicht-Lichtleiter (3) und einem Dünnschicht-Heizelement (4), wobei der Dünnschicht-Lichtleiter (3) und das Dünnschicht-Heizelement (4) entweder auf einander gegenüberliegenden Seiten der Trägerschicht (2) oder auf derselben Seite der Trägerschicht (2) angeordnet sind, **dadurch gekennzeichnet, dass** der Chip (1) des Weiteren einen Dünnschicht-Temperatursensor aufweist, der in den Chip (1) integriert ist.

2. Opto-elektronischer Chip (1) nach Anspruch 1, **dadurch gekennzeichnet, dass** die Trägerschicht (2) vollständig oder zumindest teilweise aus einem opaken oder transparenten Material, vorzugsweise aus Si oder einem SiO2 basiertem Glas oder Kristall, besteht.

3. Opto-elektronischer Chip (1) nach Anspruch 1 oder 2, **dadurch gekennzeichnet, dass** sich zwischen der Trägerschicht (2) und dem Dünnschichtwellenleiter (3) eine weitere transparente Schicht (5) befindet, die einen niedrigeren Brechungsindex als die Trägerschicht (2), vorzugsweise einen Brechungsindex zwischen 1.2 und 1.5, aufweist.

4. Opto-elektronischer Chip (1) nach Anspruch 3, **dadurch gekennzeichnet, dass** die weitere transparente Schicht (5) aus einem Polymer oder einem amorphen oder kristallinen Material besteht.

5. Opto-elektronischer Chip (1) nach einem der vorhergehenden Ansprüche, **dadurch gekennzeichnet, dass** Heizelement (4) optisch transparent ist und / oder aus einer Indium-Zinn-Oxid Verbindung besteht.

6. Opto-elektronischer Chip (1) nach einem der vorhergehenden Ansprüche, **dadurch gekennzeichnet, dass** das Dünnschicht-Heizelement (4) ein Widerstandsheizelement ist oder ein solches umfasst.

7. Opto-elektronischer Chip (1) nach einem der vorhergehenden Ansprüche, **dadurch gekennzeichnet, dass** weiterhin eine Sensor-Schicht (7) vorgesehen ist, welche vorzugsweise Metall aufweist und / oder aus Metall besteht und welche vorzugsweise eine Außenoberfläche des optoelektronischen Chips (1) zumindest teilweise bedeckt und weiterhin vorzugsweise dazu ausgelegt ist, mit einer Probe (8) in thermischen Kontakt zu treten.

8. Opto-elektronischer Chip (1) nach Anspruch 7, dadaurch gekennzteichnet, dass eine Temperaturregulierung mittels eines Feedback-Systems zwischen der Sensor-Schicht (7) und dem Heizelement (4) erfolgt.

9. Opto-elektronischer Chip (1) nach einem der vorhergehenden Ansprüche, **dadurch gekennzeichnet, dass** eine Außenoberfläche des optoelektronischen Chips (1), welche dazu ausgelegt ist, mit einer Probe (8) in Kontakt zu treten, zumindest teilweise oder vollständig eine Oberflächenmodifikation und / oder Oberflächenfunktionalisierung aufweist, um in der Probe (8) enthaltene Moleküle, insbesondere biologische Moleküle, zu binden.

10. Verwendung eines opto-elektronischen Chips (1) nach einem der vohergehenden Ansprüche zur Aufnahme einer Probe (8) bei der Visualisierung temperatursensitiver Prozesse, wobei eine Probe (8), vorzugsweise eine zumindest teilweise flüssige, feste oder gelförmige Probe (8) derart auf den opto-elektronischen Chip (1) aufgebracht wird, dass die Probe (8) den Dünnschicht-Lichtleiter (3) teilweise oder vollständig umgibt.

11. Verwendung nach Anspruch 10, dadurch gekenzeichnet, dass die Probe (8) mindestens einen oder eine Mehrzahl an Partikel(n) (10) enthält, welche(r) dazu fähig und / oder ausgelegt ist / sind, mit einem geführten Modus des Dünnschicht- Lichtleiters (3) in Wechselwirkung zu treten.

## Claims

1. Optoelectronic chip (1) for holding a sample (8) during the visualization of temperature-dependent processes using a microscope, comprising a carrier layer (2), a thin-film light guide (3), and a thin-film heating element (4), wherein the thin-film light guide (3) and the thin-film heating element (4) are arranged either on opposite sides of the substrate layer (2) or on the same side of the substrate layer (2), **characterized in** th , the chip (1) further comprises a thin-film temperature sensor that is integrated into the chip (1).

2. An optoelectronic chip (1) according to claim 1, **characterized in that** the carrier layer (2) consists entirely or at least partially of an opaque or transparent material, preferably Si or an SiO₂-based glass or crystal.

3. An optoelectronic chip (1) according to claim 1 or 2, **characterized in that** a further transparent layer (5) is located between the substrate layer (2) and the thin-film waveguide (3), which has a refractive index that is lower than that of the substrate layer (2), preferably an index of refraction between 1.2 and 1.5.

4. Optoelectronic chip (1) according to claim 3, **characterized in that** the additional transparent layer (5) consists of a polymer or an amorphous or crystalline material.

5. An optoelectronic chip (1) according to one of the preceding claims, **characterized in that** the heating element (4) is optically transparent and/or consists of an indium-tin oxide compound.

6. An optoelectronic chip (1) according to one of the preceding claims, **characterized in that** the thin-film heating element (4) is a resistive heating element or comprises such an element.

7. An optoelectronic chip (1) according to one of the preceding claims, **characterized in that** a sensor layer (7) is further provided, which preferably comprises metal and/or consists of metal, and which preferably at least partially covers an outer surface of the optoelectronic chip (1) and is further preferably designed to come into thermal contact with a sample (8).

8. An optoelectronic chip (1) according to claim 7, **characterized in that** temperature regulation is achieved by means of a feedback system between the sensor layer (7) and the heating element (4).

9. An optoelectronic chip (1) according to one of the preceding claims, **characterized in that** an outer surface of the optoelectronic chip (1), which is designed to come into contact with a sample (8), has, at least partially or completely, undergone surface modification and/or surface functionalization in order to bind molecules, in particular biological molecules, contained in the sample (8).

10. Use of an optoelectronic chip (1) according to one of the preceding claims for receiving a sample (8) in the visualization of temperature-sensitive processes, wherein a sample (8), preferably an at least partially liquid, solid, or gel-like sample (8), is applied to the optoelectronic chip (1) in such a way that the sample (8) partially or completely surrounds the thin-film optical fiber (3).

11. Use according to claim 10, **characterized in that** the sample (8) contains at least one or a plurality of particles (10) that are capable and/or designed to interact with a guided mode of the thin-film optical waveguide (3).

## Revendications

1. Puce optoélectronique (1) destinée à recevoir un échantillon (8) lors de la visualisation de processus dépendants de la température à l'aide d'un microscope, comprenant une couche de support (2), un guide de lumière à couche mince (3) et un élément chauffant à couche mince (4), le guide de lumière à couche mince (3) et l'élément chauffant en couche mince (4) sont disposés soit sur des faces opposées de la couche de support (2), soit sur la même face de la couche de support (2), **caractérisée en ce qu** la puce (1) comporte en outre un capteur de température en couche mince qui est intégré dans la puce (1).

2. Puce optoélectronique (1) selon la revendication 1, **caractérisée en ce que** la couche de support (2) est constituée entièrement ou au moins partiellement d'un matériau opaque ou transparent, de préférence de Si ou d'un verre ou d'un cristal à base de SiO₂.

3. Puce optoélectronique (1) selon la revendication 1 ou 2, **caractérisée en ce qu'**une couche transparente supplémentaire (5) est disposée entre la couche de support (2) et le guide d'ondes à couche mince (3), laquelle présente un indice de réfraction ement inférieur à celui de la couche de support (2), de préférence compris entre 1,2 et 1,5.

4. Puce optoélectronique (1) selon la revendication 3, **caractérisée en ce que** la couche transparente supplémentaire (5) est constituée d'un polymère ou d'un matériau amorphe ou cristallin.

5. Puce optoélectronique (1) selon l'une des revendications précédentes, **caractérisée en ce que** l'élément chauffant (4) est optiquement transparent et/ou est constitué d'un composé d'oxyde d'indium-étain.

6. Puce optoélectronique (1) selon l'une des revendications précédentes, **caractérisée en ce que** l'élément chauffant à couche mince (4) est un élément chauffant à résistance ou en comprend un.

7. Puce optoélectronique (1) selon l'une des revendications précédentes, **caractérisée en ce qu'**elle comporte en outre une couche de capteur (7) qui comprend de préférence du métal et/ou est constituée de métal, et qui recouvre de préférence au moins partiellement une surface extérieure de la puce optoélectronique (1) et est en outre de préférence conçue pour entrer en contact thermique avec un échantillon (8).

8. Puce optoélectronique (1) selon la revendication 7, **caractérisée en ce qu'**une régulation de la température s'effectue au moyen d'un système de rétroaction entre la couche de capteur (7) et l'élément chauffant (4).

9. Puce optoélectronique (1) selon l'une des revendications précédentes, **caractérisée en ce qu'**une surface extérieure de la puce optoélectronique (1), qui est conçue pour entrer en contact avec un échantillon (8), présente, au moins partiellement ou entièrement, une modification de surface et/ou une fonctionnalisation de surface afin de lier des molécules contenues dans l'échantillon (8), en particulier des molécules biologiques.

10. Utilisation d'une puce optoélectronique (1) selon l'une des revendications précédentes pour recevoir un échantillon (8) lors de la visualisation de processus sensibles à la température, dans laquelle un échantillon (8), de préférence un échantillon (8) au moins partiellement liquide, solide ou gélatineux, est déposé sur la puce optoélectronique (1) de telle sorte que l'échantillon (8) entoure partiellement ou entièrement le guide de lumière à couche mince (3).

11. Utilisation selon la revendication 10, **caractérisée en ce que** l'échantillon (8) contient au moins une ou plusieurs particules (10) qui sont capables et/ou conçues pour interagir avec un mode guidé du guide d'onde à couche mince (3).
